(19) Europäisches Patentamt
European Patent Office
Office européen des brevets

(11) EP 1 460 061 A1

(12) **EUROPEAN PATENT APPLICATION**
published in accordance with Art. 158(3) EPC

(43) Date of publication:
**22.09.2004 Bulletin 2004/39**

(21) Application number: **02786071.7**

(22) Date of filing: **10.12.2002**

(51) Int Cl.7: **C07D 207/14**, C07D 211/56

(86) International application number:
**PCT/JP2002/012895**

(87) International publication number:
**WO 2003/055858 (10.07.2003 Gazette 2003/28)**

(84) Designated Contracting States:
**AT BE BG CH CY CZ DE DK EE ES FI FR GB GR IE IT LI LU MC NL PT SE SI SK TR**

(30) Priority: **27.12.2001 JP 2001397551**

(71) Applicant: **Toray Fine Chemicals Co., Ltd.**
**Urayasu-shi, Chiba 279-8555 (JP)**

(72) Inventors:
- **ONO, Takae**
**Tokai-shi, Aichi 477-0032 (JP)**
- **SATO, Haruyo**
**Nagoya-shi, Aichi 454-0926 (JP)**

(74) Representative: **Coleiro, Raymond et al**
**MEWBURN ELLIS LLP**
**York House**
**23 Kingsway**
**London WC2B 6HP (GB)**

(54) **PROCESS FOR PRODUCING 1-ALKOXYCARBONYL NITROGENOUS SATURATED HETEROCYCLIC DERIVATIVE**

(57) A method for producing N-protected heterocyclic compounds such as 1-alkoxycarbonyl-3-aminopyrrolidines through position-selective reaction at the nitrogen atom that constitutes the hetero ring of a nitrogen-containing saturated heterocyclic compound having two nitrogen atoms such as 3-aminopyrrolidine. A dialkyl dicarbonate (ROCO-O-COOR) is reacted with a nitrogen-containing saturated heterocyclic compounds having two nitrogen atoms, at pH of from 9 to 14 to obtain a high-purity 1-alkoxycarbonyl nitrogen-containing saturated heterocyclic compound.



Printed by Jouve, 75001 PARIS (FR)

## Description

TECHNICAL FIELD

The present invention relates to a method for producing 1-alkoxycarbonyl nitrogen-containing, saturated heterocyclic derivatives that are useful as intermediates for medicines and agricultural chemicals, concretely to a method for producing 1-alkoxycarbonyl-3-aminopyrrolidine derivatives through position-selective reaction of only the secondary amino group of the pyrrolidine ring of a 3-aminopyrrolidine derivative having two different amino groups, or to a method for producing 1-alkoxycarbonylaminopiperidine derivatives through position-selective reaction of only the secondary amino group of the piperidine ring of a 4-aminopiperidine derivative having two different amino groups.

BACKGROUND ART

3-Aminopyrrolidine derivatives have two different amino groups. For producing 1-alkoxycarbonyl-3-aminopyrrolidine derivatives at high yield in a mode of position-selective reaction, known is a method that comprises protecting the 3-amino group of 3-aminopyrrolidine through reaction with benzaldehyde, then reacting the 1-amino group thereof with di-tertiary butyl dicarbonate, and removing the protective group (JP-A 2000-53681).

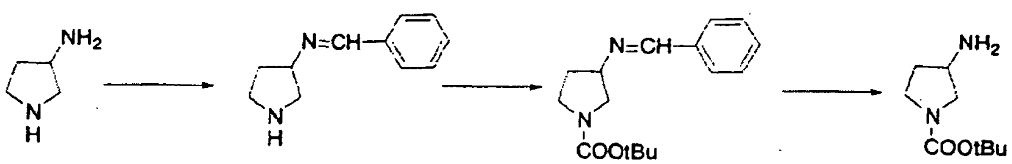

This production method is good for producing high-purity 1-alkoxycarbonyl-3-aminopyrrolidines, but is defective in that its reaction process is long and its yield is low since the hydrolysis of the Schiff base therein is accompanied by the hydrolysis of the tertiary butoxycarbonyl group in the intermediate. If 3-aminopyrrolidine is directly reacted with di-tertiary butyl dicarbonate, then the 3-amino group thereof is also tertiary butoxycarbonylated to give an isomer as a by-product. Removing the isomer requires a complicated operation, and it is impossible to produce high-purity 1-tertiary butoxycarbonyl-3-aminopyrrolidine at high yield. Similarly, if 4-aminopiperidine is directly reacted with di-tertiary butyl dicarbonate, then the amino group thereof is also tertiary butoxycarbonylated to give an isomer as a by-product, and it is impossible to produce high-purity 1-tertiary butoxycarbonyl-4-aminopiperidine at high yield.

Therefore desired is a simple method of producing high-purity 1-alkoxycarbonyl-3-aminopyrrolidine derivatives and high-purity 1-alkoxycarbonyl-4-aminopiperidine derivatives at high yield.

We, the present inventors have assiduously investigated a simple and industrial method for producing 1-alkoxycarbonyl nitrogen-containing heterocyclic derivatives at high yield, and, as a result, have concretely found that, when a 3-aminopyrrolidine derivative is reacted with a dialkyl dicarbonate at pH of from 9 to 14, then the intended product, 1-alkoxycarbonyl-3-aminopyrrolidine derivative can be produced with good selectivity. On the basis of this finding, we have completed the present invention.

DISCLOSURE OF THE INVENTION

The invention is a method for producing 1-alkoxycarbonyl nitrogen-containing, saturated heterocyclic derivatives of a general formula (3):

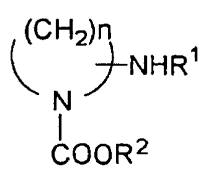

(3)

wherein $R^1$ represents a hydrogen atom, or a linear or branched alkyl group having from 1 to 10 carbon atoms; $R^2$ represents a linear or branched alkyl group having from 1 to 5 carbon atoms;
n stands for an integer of 4 to 7,
by reacting a nitrogen-containing saturated heterocyclic compound of a general formula (1):

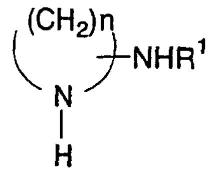

(1)

wherein $R^1$ and n have the same meanings as above,
with a dialkyl dicarbonate of a general formula (2):

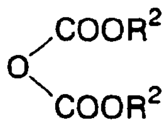

(2)

wherein $R^2$ has the same meaning as above,
at pH of from 9 to 14.

In a mode of more specifically defining the compounds therein, the invention is a method for producing 1-alkoxycarbonyl-3-aminopyrrolidine derivatives of a general formula (5):

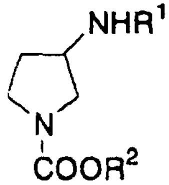

(5)

wherein $R^1$ represents a hydrogen atom, or a linear or branched alkyl group having from 1 to 10 carbon atoms; $R^2$ represents a linear or branched alkyl group having from 1 to 5 carbon atoms, by reacting a 3-aminopyrrolidine derivative of a general formula (4):

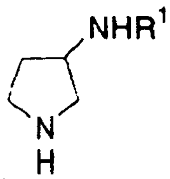

(4)

wherein $R^1$ has the same meaning as above,
with a dialkyl dicarbonate of a general formula (2):

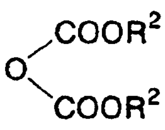

(2)

wherein $R^2$ has the same meaning as above,
at pH of from 9 to 14.

In another embodiment thereof, the invention is a method for producing 1-alkoxycarbonyl aminophenylpiperidine derivatives of a general formula (7):

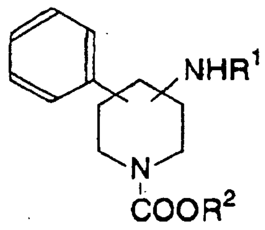

(7)

wherein $R^1$ represents a hydrogen atom, or a linear or branched alkyl group having from 1 to 10 carbon atoms; $R^2$ represents a linear or branched alkyl group having from 1 to 5 carbon atoms, by reacting an aminophenylpiperidine derivative of a general formula (6):

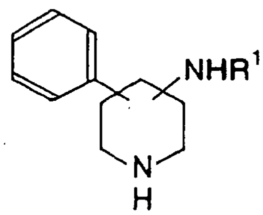

(6)

wherein $R^1$ has the same meaning as above,
with a dialkyl dicarbonate of a general formula (2):

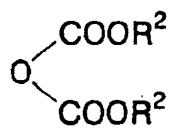

(2)

wherein $R^2$ has the same meaning as above,
at pH of from 9 to 14.

BEST MODES OF CARRYING OUT THE INVENTION

The nitrogen-containing saturated heterocyclic compounds of formula (1) that are used as the starting substance in the invention typically include the 3-aminopyrrolidine derivatives of formula (4), and in addition to these, further include 3-aminopiperidine, 3-methylaminopiperidine, 3-ethylaminopiperidine, 3-propylaminopiperidine, 3-butylaminopiperidine, 4-aminopiperidine, aminoperhydroazepine, methylaminoperhydroazepine, aminoperhydroazocine, methylaminoperhydroazocine. The 3-aminopyrrolidine derivatives of formula (4) include 3-aminopyrrolidine, 3-methylaminopyrrolidine, 3-ethylaminopyrrolidine, 3-propylaminopyrrolidine, 3-butylaminopyrrolidine. In the nitrogen-containing saturated heterocyclic compounds of formula (1), the hydrogen atom bonding to the carbon atom that constitutes the saturated hetero ring may be substituted with a phenyl group. One typical example of such phenyl group-substituted, nitrogen-containing saturated heterocyclic compounds is 3-amino-2-phenylpiperidine. The compounds may be in any form of S-configurational or R-configurational optical-active compounds, or racemates.

For the other starting material, dialkyl dicarbonates of formula (2), preferred are diethyl dicarbonate and di-tertiary butyl dicarbonate, and more preferred is di-tertiary butyl dicarbonate. The amount of the dialkyl dicarbonate to be used is preferably from 0.6 to 1.05 molar times, more preferably from 0.7 to 1.02 molar times the nitrogen-containing saturated heterocyclic compound. Within the range, the side product will be small, the reaction yield will be high, and the production efficiency will be high.

The reaction solvent may be any one not interfering with the reaction. Preferred are alcohols such as methanol, ethanol; hydrocarbons such as hexane, cyclohexane, benzene, toluene; ethers such as diethyl ether, tetrahydrofuran; chlorine compounds such as dichloromethane, chloroform; and acetonitrile. More preferred are methanol and ethanol. Also usable is a mixed solvent with water. Preferably, the amount of the solvent to be used is from 3 to 15 times by

weight, more preferably from 4 to 10 times by weight the nitrogen-containing saturated heterocyclic compound. Within the range, the reaction will be good, and the production efficiency will be high.

For reacting the two, the nitrogen-containing saturated heterocyclic compound is dissolved in a solvent, and a dialkyl dicarbonate is dropwise added thereto with stirring at a predetermined temperature and at a controlled pH of from 9 to 14, preferably from 11 to 14. For controlling the pH of the reaction system, an alkali metal hydroxide or an alkaline earth metal hydroxide may be used preferably. More preferably, sodium hydroxide or potassium hydroxide may be sued. Any of these may be fed to the reaction system while it is solid or it is in an aqueous or alcoholic solution thereof. More preferably, its alcoholic solution is added thereto.

The reaction temperature preferably falls between -20 and 40°C, more preferably between 10 and 20°C. Within the range, the position selectivity of the product is high. The reaction time varies depending on the reaction condition. In general, the system is reacted for 1 to 5 hours after the dropwise addition of dialkyl dicarbonate thereto. Thus obtained, the 1-alkoxycarbonyl nitrogen-containing, saturated heterocyclic derivative may be isolated in any ordinary manner. For example, after completion of reaction, the reaction mixture is concentrated to remove the solvent from it, and a solvent capable of dissolving the 1-alkoxycarbonyl nitrogen-containing, saturated heterocyclic derivative but not dissolving inorganic substances, for example, a toluene solvent is added to it. Then, the inorganic substances are filtered away, and the filtrate is concentrated and distilled under reduced pressure. Thus obtained, the 1-alkoxycarbonyl nitrogen-containing, saturated heterocyclic derivative contains at most 1 % of its positional isomer, 3-alkoxycarbonyl nitrogen-containing, saturated heterocyclic derivative.

When an optical-active 3-amino saturated heterocyclic compound is used in the present production method, the optical purity of the product, 1-alkoxycarbonyl-3-amino saturated heterocyclic derivative lowers little.

The invention is described in more detail with reference to the following Examples, to which, however, the invention is not limited.

In the Examples, the composition analysis of the reaction liquid and the purity analysis of the distilled matter are quantified from area % in GC. The optical analysis method varies depending on the matter to be analyzed. For example, 3-aminopyrrolidine is reacted with an optical-active tartaric acid derivative anhydride (by Toray) to give an optical-active tartaric acid derivative according to the following reaction formula, and it is analyzed through HPLC with ODS columns.

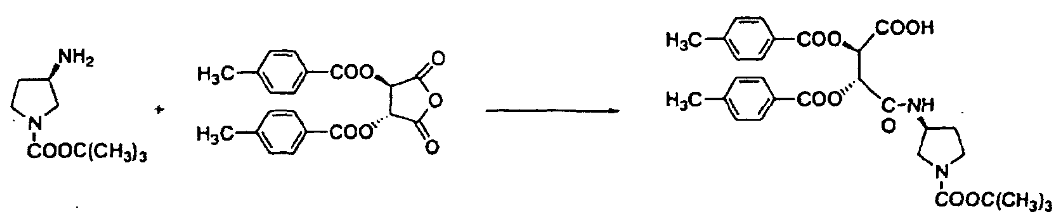

Optical Purity Calculation (for (R) > (S)):

$$\{[(R)\text{ peak area} - (S)\text{ peak area}]/[(R)\text{ peak area} + (S)\text{ peak area})\}$$

$$\times 100\ (\%\ e.e.)$$

Example 1:

60 g of methanol and 8.6 g (0.1 mols) of (S)-3-aminopyrrolidine (Chemical purity 99.8 %, optical purity 99.5 % ee - hereinafter referred to as S-AP) were fed into a 200-ml 4-neck flask equipped with a stirrer, a pH sensor and two strapped dropping funnels, and stirred at 15 to 20°C. 21.8 g (0.1 mols) of di-tertiary butyl dicarbonate (by Tokyo Chemical) was fed into it via one dropping funnel, and 25 g (0.11 mols) of methanolic solution of 25 % potassium hydroxide was thereinto via the other dropping funnel. With stirring at 15 to 20°C, di-tertiary butyl dicarbonate was dropwise added to the reaction system over a period of about 1 hour. During this, the methanolic solution of 25 % potassium hydroxide was dropwise added thereto so as to make the reaction system have a pH of from 11.8 to 12.2. The reaction mixtures were analyzed for its composition, and it comprised 4 % of the starting 3-aminopyrrolidine, 86.7 % of the product, 3-amino-1-tertiary butoxycarbonylpyrrolidine (hereinafter referred to as 1-BocAP), and 8.4 % of 1-tertiary butoxycarbonyl-3-tertiary butoxycarbonylaminopyrrolidine (hereinafter referred to as DiBocAP). After the reaction,

the reaction mixtures were concentrated under reduced pressure to make 32 g. 100 g of toluene was added to the concentrated solution, and inorganic salts were precipitated with stirring. The precipitated crystals were filtered away, and the filtrate was distilled under reduced pressure to obtain a fraction at 120 to 125°C/0.7 kPa, (S)-3-amino-1-tertiary butoxycarbonylpyrrolidine. Its weight was 15.2 g, and its yield was 81.7 %. (Chemical purity 99.1 %; optical purity 99.5 % ee. ) The positional isomer, 3-tertiary butoxycarbonylaminopyrrolidine was 0.4 %.

Example 2:

The compounds were reacted in the same manner as in Example 1, except that the amount of di-tertiary butyl dicarbonate was changed to 15.3 g (0.07 mols) and that of methanol was to 80 g. The reaction mixtures comprised 11.8 % of the starting 3-aminopyrrolidine, 85.4 % of the product, 3-amino-1-tertiary butoxycarbonylpyrrolidine, and 2.0 % of 1-tertiary butoxycarbonyl-3-tertiary butoxycarbonylaminopyrrolidine.

In the same manner as in Example 1, this was distilled under reduced pressure to give a fraction at 70 to 80°C/2.6 kPa, a fore-running consisting essentially of the starting 3-aminopyrrolidine. Its weight was 1.2 g, and its recovery was 13.9%. The distillation also gave a fraction at 120 to 125°C/0.7 kPa, (S)-3-amino-1-tertiary butoxycarbonylpyrrolidine. Its weight was 14.9 g, and its yield was 80.7 %. (Chemical purity 99.5 %; optical purity 99.5 % ee.) The fore-running is (S)-3-aminopyrrolidine with minor (S)-3-amino-1-tertiary butoxycarbonylpyrrolidine, and it may be recovered and refused as the material for the reaction.

Examples 3 to 6, Comparative Example 1:

In the process of Example 1, the pH of the reaction system was varied.

In Comparative Example 1, the pH of the reaction system was not controlled at all.

[Table 1]

| | pH of Reaction System | Composition of Reaction Mixture | | |
|---|---|---|---|---|
| | | S-AP | 1-BocAP | DiBocAP |
| Example 3 | 10.0 | 5.9 | 78.4 | 14.7 |
| Example 4 | 11.0 | 4.3 | 58.9 | 8.8 |
| Example 5 | 13.0 | 4.8 | 86.9 | 7.7 |
| Example 6 | 14.0 | 8.1 | 83.4 | 7.7 |
| Comparative Example 1 | * | 13.8 | 49.5 | 35.7 |

* At the start of the reaction, the pH of the reaction system was 12.5. With the reaction going on, the pH lowered and it was 7.7 at the end of the reaction.

Example 7:

The compounds were reacted in the same manner as in Example 1, for which, however, the reaction temperature was 30 to 35°C. The reaction mixtures comprised 4.1 % of AP, 88.3 % of 1-BocAP, and 7.0 % of DiBocAP.

Example 8:

90 g of ethanol and 10.0 g (0.1 mols) of 3-methylaminopyrrolidine were fed into a 200-ml 4-neck flask equipped with a stirrer, a pH sensor and two strapped dropping funnels, and stirred at 10 to 15°C. 21.8 g (0.1 mols) of di-tertiary butyl dicarbonate was fed into it via one dropping funnel, and 17.6 g (0.11 mols) of aqueous solution of 25 % sodium hydroxide was thereinto via the other dropping funnel. With stirring at 10 to 15°C, di-tertiary butyl dicarbonate was dropwise added to the reaction system over a period of about 1 hour. During this, the aqueous solution of 25 % sodium hydroxide was dropwise added thereto so as to make the reaction system have a pH of from 11.8 to 12.2. The reaction mixtures were analyzed for its composition, and it comprised 4 % of the starting 3-methylaminopyrrolidine, 93.2 % of the product, 3-methylamino-1-tertiary butoxycarbonylpyrrolidine, and 0.5 % of 1-tertiary butoxycarbonyl-3-tertiary butoxycarbonylmethylaminopyrrolidine.

Example 9:

90 g of ethanol and 10.0 g (0.1 mols) of 3-aminopiperidine were fed into a 200-ml 4-neck flask equipped with

a stirrer, a pH sensor and two strapped dropping funnels, and stirred at 10 to 15°C. 21.8 g (0.1 mols) of di-tertiary butyl dicarbonate was fed into it via one dropping funnel, and 17.6 g (0.11 mols) of aqueous solution of 25 % sodium hydroxide was thereinto via the other dropping funnel. With stirring at 10 to 15°C, di-tertiary butyl dicarbonate was dropwise added to the reaction system over a period of about 1 hour. During this, the aqueous solution of 25 % sodium hydroxide was dropwise added thereto so as to make the reaction system have a pH of from 11.8 to 12.2. The reaction mixtures were analyzed for its composition, and it comprised 6 % of the starting 3-aminopiperidine, 91.5 % of the product, 3-amino-1-tertiary butoxycarbonylpiperidine, and 1.1 % of 3-tertiary butoxycarbonylaminopiperidine.

Comparative Example 2:

The compounds were reacted in the same manner as in Example 9, for which, however, the pH of the reaction system was not controlled. The reaction mixtures were analyzed, and it comprised 25 % of the starting 3-aminopiperidine, 69.2 % of the product, 3-amino-1-tertiary butoxycarbonylpiperidine, and 4.1 % of 3-tertiary butoxycarbonylaminopiperidine. The reaction speed was low, and the selectivity was low.

Example 10:

120 g of ethanol and 17.6 g (0.1 mols) of 3-amino-2-phenylpiperidine were fed into a 200-ml 4-neck flask equipped with a stirrer, a pH sensor and two strapped dropping funnels, and stirred at 10 to 15°C. 21.8 g (0.1 mols) of di-tertiary butyl dicarbonate was fed into it via one dropping funnel, and 17.6 g (0.11 mols) of aqueous solution of 25 % sodium hydroxide was thereinto via the other dropping funnel. With stirring at 10 to 15°C, di-tertiary butyl dicarbonate was dropwise added to the reaction system over a period of about 2 hours. During this, the aqueous solution of 25 % sodium hydroxide was dropwise added thereto so as to make the reaction system have a pH of from 11.8 to 12.2, and the reaction was continued for further 1 hour. The reaction mixtures were analyzed for its composition, and it comprised 31 % of the starting 3-amino-2-phenylpiperidine, 62.8 % of the product, 3-amino-2-phenyl-1-tertiary butoxycarbonylpiperidine, and 0.2 % of 2-phenyl-3-tertiary butoxycarbonylaminopiperidine.

INDUSTRIAL APPLICABILITY

According to the invention, high-purity 1-alkoxycarbonyl nitrogen-containing, saturated heterocyclic derivatives can be produced in one step from nitrogen-containing saturated heterocyclic compounds.

## Claims

**1.** A method for producing 1-alkoxycarbonyl nitrogen-containing, saturated heterocyclic derivatives of a general formula (3):

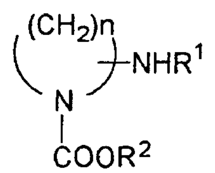

(3)

wherein $R^1$ represents a hydrogen atom, or a linear or branched alkyl group having from 1 to 10 carbon atoms;
$R^2$ represents a linear or branched alkyl group having from 1 to 5 carbon atoms;
n stands for an integer of 4 to 7,
by reacting a nitrogen-containing saturated heterocyclic compound of a general formula (1):

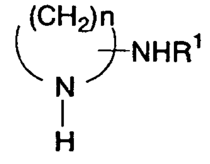

(1)

wherein $R^1$ and n have the same meanings as above, with a dialkyl dicarbonate of a general formula (2):

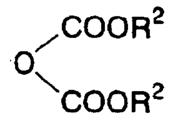

(2)

wherein $R^2$ has the same meaning as above, at pH of from 9 to 14.

**2.** The method for producing 1-alkoxycarbonyl nitrogen-containing, saturated heterocyclic derivatives as claimed in claim 1, wherein the reaction solvent is alcohol.

**3.** The method for producing 1-alkoxycarbonyl nitrogen-containing, saturated heterocyclic derivatives as claimed in claim 1 or 2, wherein the compound of formula (2) is dibutyl dicarbonate.

**4.** The method for producing 1-alkoxycarbonyl nitrogen-containing, saturated heterocyclic derivatives as claimed in claim 1 or 2, wherein the compound of formula (1) is 3-aminopiperidine.

**5.** The method for producing 1-alkoxycarbonyl nitrogen-containing, saturated heterocyclic derivatives as claimed in claim 1 or 2, wherein the compound of formula (1) is optical-active 3-aminopiperidine, the compound of formula (2) is di-tertiary butyl dicarbonate, and the compound of formula (3) is 1-tertiary butoxycarbonyl-3-aminopiperidine.

**6.** A method for producing 1-alkoxycarbonyl-3-aminopyrrolidine derivatives of a general formula (5):

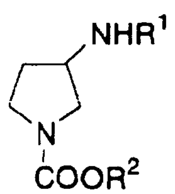

(5)

wherein $R^1$ represents a hydrogen atom, or a linear or branched alkyl group having from 1 to 10 carbon atoms; $R^2$ represents a linear or branched alkyl group having from 1 to 5 carbon atoms, by reacting a 3-aminopyrrolidine derivative of a general formula (4):

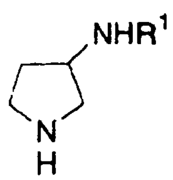

(4)

wherein $R^1$ has the same meaning as above, with a dialkyl dicarbonate of a general formula (2):

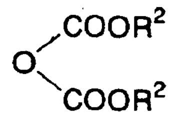

(2)

wherein $R^2$ has the same meaning as above, at pH of from 9 to 14.

**7.** The method for producing 1-alkoxycarbonyl-3-aminopyrrolidine derivatives as claimed in claim 6, wherein the reaction solvent is alcohol.

**8.** The method for producing 1-alkoxycarbonyl-3-aminopyrrolidine derivatives as claimed in claim 6 or 7, wherein the compound of formula (2) is dibutyl dicarbonate.

**9.** The method for producing 1-alkoxycarbonyl-3-aminopyrrolidine derivatives as claimed in claim 6 or 7, wherein the compound of formula (4) is 3-aminopyrrolidine.

**10.** The method for producing 1-alkoxycarbonyl-3-aminopyrrolidine derivatives as claimed in claim 6 or 7, wherein the compound of formula (4) is optical-active 3-aminopyrrolidine, the compound of formula (2) is di-tertiary butyl dicarbonate, and the compound of formula (5) is 1-tertiary butoxycarbonyl-3-aminopyrrolidine.

**11.** A method for producing 1-alkoxycarbonyl-aminophenylpiperidine derivatives of a general formula (7):

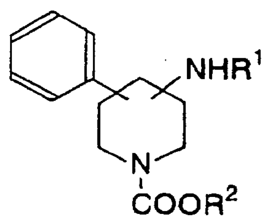

(7)

wherein $R^1$ represents a hydrogen atom, or a linear or branched alkyl group having from 1 to 10 carbon atoms; $R^2$ represents a linear or branched alkyl group having from 1 to 5 carbon atoms,
by reacting an aminophenylpiperidine derivative of a general formula (6):

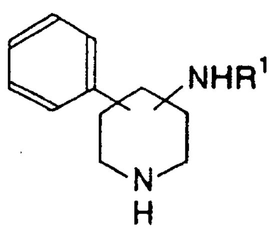

(6)

wherein $R^1$ has the same meaning as above,
with a dialkyl dicarbonate of a general formula (2):

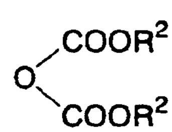

(2)

wherein $R^2$ has the same meaning as above,
at pH of from 9 to 14.

**12.** The method for producing 1-alkoxycarbonyl-aminophenylpiperidine derivatives as claimed in claim 11, wherein the compound of formula (6) is 3-amino-2-phenylpiperidine, the compound of formula (2) is di-tertiary butyl dicarbonate, and the compound of formula (7) is 1-tertiary butoxycarbonyl-3-amino-2-phenylpiperidine.

# INTERNATIONAL SEARCH REPORT

International application No.
PCT/JP02/12895

A. CLASSIFICATION OF SUBJECT MATTER
Int.Cl[7] C07D207/14, 211/56

According to International Patent Classification (IPC) or to both national classification and IPC

B. FIELDS SEARCHED

Minimum documentation searched (classification system followed by classification symbols)
Int.Cl[7] C07D207/14, 211/56

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| Y | JP 2000-119253 A (Daiichi Pharmaceutical Co., Ltd.), 25 April, 2000 (25.04.00), Referential example 60 & EP 1104754 A | 1-12 |
| Y | GREENE, T. W., PROTECTIVE GROUPS IN ORGANIC SYNTHESIS, JOHN WILEY & SONS, INC., (1991), page 327 | 1-12 |
| P,A | WO 02/55494 A (Sankyo Co., Ltd.), 18 July, 2002 (18.07.02), Full text & JP 2002-275155 A | 1-12 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 January, 2003 (08.01.03) | 21 January, 2003 (21.01.03) |
| Name and mailing address of the ISA/ Japanese Patent Office | Authorized officer |
| Facsimile No. | Telephone No. |

Form PCT/ISA/210 (second sheet) (July 1998)